# EUROPEAN PATENT APPLICATION

(11) **EP 3 978 435 A1**
(43) Date of publication of application: **06.04.2022**
(21) Application number: 20812583.1
(22) Date of filing: 03.04.2020
(51) Int. Cl.: C01B 37/00, C07D 301/19, B01J 37/04, B01J 37/08, C07D 303/04, B01J 29/03

(54) **METHOD FOR PRODUCING TITANIUM-CONTAINING SILICON OXIDE, METHOD FOR PRODUCING EPOXIDE, AND TITANIUM-CONTAINING SILICON OXIDE**

(30) Priority: 29.05.2019 JP 2019100506
(71) Applicant: SUMITOMO CHEMICAL COMPANY LIMITED, Chuo-ku Tokyo 104-8260 (JP)
(72) Inventor: MATOBA Motoshi, Ichihara-shi, Chiba 299-0195 (JP)
(74) Representative: Winter, Brandl - Partnerschaft mbB
(86) International application number: PCT/JP2020/015413
(87) International publication number: WO 2020/241052

(57) **Abstract**

A method for producing an epoxide using a titanium-containing silicon oxide catalyst that can be produced using a mold agent that is a mixture of a specific quaternary ammonium compound and a specific tertiary amine compound.

## Description

### TECHNICAL FIELD

The present invention relates to a method for producing a titanium-containing silicon oxide, a method for producing an epoxide from an olefin using a titanium-containing silicon oxide produced by the method as a catalyst, and a titanium-containing silicon oxide.

### BACKGROUND ART

A method for producing an epoxide from a hydroperoxide and an olefin in the presence of a catalyst is known. As a catalyst used in this method, for example, Patent Document 1 describes a titanium-containing silicon oxide obtained by a production method comprising a first step of mixing a silica source, a titanium source and a template agent in a liquid state to obtain a solid containing a catalyst component and a template agent, a second step of operating a solvent extraction to remove the template agent from the solid obtained in the first step, a third step of replacing an extraction solvent included in the solid after removing the template agent obtained in the second step by a solvent that is substantially inactive to a silylating agent used in a fourth step described below, and a fourth step of subjecting the solid obtained in the third step to a silylation treatment to obtain a silylated catalyst.

### PRIOR ART DOCUMENTS

### PATENT DOCUMENT

**Patent Document 1:** JP 2004-195379

### SUMMARY OF INVENTION

### TECHNICAL PROBLEM

In a reaction of generating an epoxide from an olefin and a hydroperoxide, a method capable of producing an epoxide in a high yield has been desired.

### SOLUTION TO PROBLEM

The present invention relates to the following but is not limited thereto.

### [Invention 1]

A method for producing a titanium-containing silicon oxide, comprising the following steps:
a step of mixing a silicon source, a template agent and a solvent to obtain a solid including a silicon oxide and a template agent (raw material mixing step);
a step of removing the template agent from the solid obtained in the raw material mixing step to obtain a solid including a silicon oxide (template agent removing step);
a step of contacting the solid obtained in the template agent removing step with a silylating agent to obtain a solid including a silylated silicon oxide (silylation step); and a step of introducing titanium into a system (titanium introducing step), wherein
the template agent is a mixture of a quaternary ammonium compound having a quaternary ammonium ion represented by the following formula I and a tertiary amine compound represented by the following formula II,

   [NR¹R²R³R⁴]⁺ I

wherein
   R¹ represents a hydrocarbon group having 2 to 36 carbon atoms; and
   R², R³ and R⁴ each independently represent a hydrocarbon group having 1 to 6 carbon atoms, and

      NR⁵R⁶R⁷ II
wherein
   R⁵ represents a hydrocarbon group having 2 to 36 carbon atoms; and
   R⁶ and R⁷ each independently represent a hydrogen atom or a hydrocarbon group having 1 to 6 carbon atoms; and
   a molar ratio of the tertiary amine compound to the quaternary ammonium compound is from 0.005 to 0.16.

### [Invention 2]

The method according to Invention 1, wherein R¹ is a hydrocarbon group having 10 to 22 carbon atoms.

### [Invention 3]

The method according to Invention 1 or 2, wherein R⁵ is a hydrocarbon group having 10 to 22 carbon atoms.

### [Invention 4]

The method according to any one of Inventions 1 to 3, wherein R¹ and R⁵ are the same as each other.

### [Invention 5]

The method according to Invention 1, wherein the quaternary ammonium ion is hexadecyltrimethylammonium ion, and
the tertiary amine compound is hexadecyldimethylamine.

### [Invention 6]

The method according to any one of Inventions 1 to 5, wherein a molar ratio of the tertiary amine compound to the quaternary ammonium compound is from 0.01 to 0.13.

### [Invention 7]

The method according to Invention 6, wherein a molar ratio of the tertiary amine compound to the quaternary ammonium compound is from 0.04 to 0.13.

### [Invention 8]

The method according to any one of Inventions 1 to 7, wherein the raw material mixing step is the following step and the titanium introducing step is carried out in the raw material mixing step:
the step of mixing a silicon source, a titanium source, a template agent and a solvent to obtain a solid including a silicon oxide into which titanium is introduced and a template agent.

### [Invention 9]

A titanium-containing silicon oxide producible by the method according to any one of Inventions 1 to 8.

### [Invention 10]

A method for producing an epoxide, comprising a step of reacting an olefin with a hydroperoxide in the presence of a titanium-containing silicon oxide producible by the method according to any one of Inventions 1 to 8 or the titanium-containing silicon oxide according to Invention 9.

### [Invention 11]

The method according to Invention 10, wherein the olefin is an α-olefin.

### [Invention 12]

The method according to Invention 10, wherein the olefin is propylene.

### [Invention 13]

The method according to any one of Inventions 10 to 12, wherein the hydroperoxide is cumene hydroperoxide.

### ADVANTAGEOUS EFFECT OF INVENTION

According to one aspect of the present invention, in a reaction of generating an epoxide from an olefin and a hydroperoxide, a method for producing an epoxide in a high yield, and the like are provided.

### DESCRIPTION OF EMBODIMENTS

### Definitions

Herein, a term "a-olefin" means a hydrocarbon having a carbon-carbon unsaturated double bond at an α position.

Herein, a term "hydrocarbon group having X to Y carbon atoms" means a hydrocarbon group in which the number of carbon atoms is X to Y

Herein, the description of "lower limit to upper limit" representing the numerical range represents "lower limit or more and upper limit or less". That is, these descriptions represent the numerical range including a lower limit and an upper limit.

A method for producing a titanium-containing silicon oxide according to one aspect of the present invention comprises a raw material mixing step, a template agent removing step, a silylation step and a titanium introducing step.

A titanium-containing silicon oxide refers to a compound in which a part of Si in a porous silicate (SiO₂) is substituted by Ti. The compound has a bond represented by -Si-O-Ti.

### Raw material mixing step

The raw material mixing step is a step of mixing a silicon source, a template agent and a solvent to obtain a solid including a silicon oxide and a template agent and also sometimes referred to as Step A.

The "silicon source" means a silicon oxide and a silicon oxide precursor. The silicon oxide precursor means a compound in which a part or the whole of the silicon oxide precursor becomes a silicon oxide by reacting the silicon oxide precursor with water.

Examples of the silicon oxide include amorphous silica. Examples of the silicon oxide precursor include an alkoxysilane, an alkyltrialkoxysilane, a dialkyldialkoxysilane and a 1,2-bis(trialkoxysilyl)alkane. Examples of the alkoxysilane include tetramethyl orthosilicate, tetraethyl orthosilicate and tetrapropyl orthosilicate. Examples of alkyltrialkoxysilane include trimethoxy(methyl)silane. Examples of the dialkyldialkoxysilane include dimethoxydimethylsilane. As the silicon source, a single one may be used, or several kinds thereof may be used in combination.

When the silicon oxide precursor is used as the silicon source, water is preferably used as a part or the whole of the solvent in Step A. When the silicon oxide precursor is mixed with water, a part or the whole of the silicon oxide precursor is changed to the silicon oxide.

The template agent means a substance capable of forming a pore structure in the titanium-containing silicon oxide. The template agent is a mixture of a quaternary ammonium compound having a quaternary ammonium ion represented by the following formula I and a tertiary amine compound represented by the following formula II.

[NR¹R²R³R⁴]⁺ I

wherein R¹ represents a hydrocarbon group having 2 to 36 carbon atoms, and R² to R⁴ each independently represent a hydrocarbon group having 1 to 6 carbon atoms, and

   NR⁵R⁶R⁷ II
wherein R⁵ represents a hydrocarbon group having 2 to 36 carbon atoms, and R⁶ and R⁷ each independently represent a hydrogen atom or a hydrocarbon group having 1 to 6 carbon atoms.

In formula I, R¹ is a hydrocarbon group having 2 to 36 carbon atoms, and may be linear or branched and may be aliphatic or aromatic. R¹ is preferably a hydrocarbon group having 10 to 22 carbon atoms. R² to R⁴ are each independently a hydrocarbon group having 1 to 6 carbon atoms, and may be linear or branched. R² to R⁴ are preferably all methyl groups.

Specific examples of the quaternary ammonium ion represented by formula I include cations such as tetraethylammonium, tetrapropylammonium, tetrabutylammonium, decyltrimethylammonium, dodecyltrimethylammonium, hexadecyltrimethylammonium, octadecyltrimethylammonium, eicosyltrimethylammonium, behenyltrimethylammonium and benzyltrimethylammonium.

Specific examples of the compound including the quaternary ammonium ion represented by formula I include tetraethylammonium hydroxide, tetrapropylammonium hydroxide, tetrabutylammonium hydroxide, decyltrimethylammonium hydroxide, decyltrimethylammonium chloride, decyltrimethylammonium bromide, dodecyltrimethylammonium hydroxide, dodecyltrimethylammonium chloride, dodecyltrimethylammonium bromide, hexadecyltrimethylammonium hydroxide, hexadecyltrimethylammonium chloride, hexadecyltrimethylammonium bromide, octadecyltrimethylammonium hydroxide, octadecyltrimethylammonium chloride, octadecyltrimethylammonium bromide, eicosyltrimethylammonium hydroxide, eicosyltrimethylammonium chloride, eicosyltrimethylammonium bromide, behenyltrimethylammonium hydroxide, behenyltrimethylammonium chloride and behenyltrimethylammonium bromide.

In formula II, R⁵ is a hydrocarbon group having 2 to 36 carbon atoms, and may be linear or branched and may be aliphatic or aromatic. R⁵ is preferably a hydrocarbon group having 10 to 22 carbon atoms. R⁶ and R⁷ are each independently a hydrogen atom or a hydrocarbon group having 1 to 6 carbon atoms, and may be linear or branched. R⁶ and R⁷ are preferably methyl groups.

Specific examples of the amine represented by formula II include ethylamine, propylamine, butylamine, octylamine, nonylamine, decylamine, undecylamine, dodecylamine, tridecylamine, tetradecylamine, pentadecylamine, hexadecylamine, heptadecylamine, octadecylamine, nonadecylamine, eicosylamine, behenylamine and benzylamine, and methylalkylamine and dimethylalkylamine in which at least one hydrogen atom in these amines is substituted by a methyl group.

In the mixture, the molar ratio of the tertiary amine compound to the quaternary ammonium compound is 0.005 to 0.16, and in one aspect, the molar ratio thereof may be 0.005 to 0.15, 0.005 to 0.14, 0.005 to 0.13, 0.01 to 0.16, 0.01 to 0.15, 0.01 to 0.14, 0.01 to 0.13, 0.02 to 0.15, 0.02 to 0.14, 0.02 to 0.13, 0.03 to 0.16, 0.03 to 0.15, 0.03 to 0.14, 0.03 to 0.13, 0.04 to 0.16, 0.04 to 0.15, 0.04 to 0.14, or 0.04 to 0.13.

Examples of the method for adjusting the molar ratio of the tertiary amine compound to the quaternary ammonium compound in the mixture include:
a method for adjusting a mixing ratio of the quaternary ammonium compound and the tertiary amine compound;
a method for adjusting the molar ratio of the tertiary amine compound to the quaternary ammonium compound by, in the mixture of the quaternary ammonium compound and the tertiary amine compound, reacting the tertiary amine compound with an alkylating agent and converting a part of the tertiary amine compound into the quaternary ammonium compound, followed by carrying out an ion exchange if necessary; and
a method for adjusting the molar ratio of the tertiary amine compound to the quaternary ammonium compound by subjecting the mixture of the quaternary ammonium compound and the tertiary amine compound to distillation, extraction, recrystallization, chromatography or a combination of two or more of these methods.

Examples of the alkylating agent include methyl chloride, methyl bromide and methyl iodide.

The mixing of the silicon source and the template agent is carried out in the presence of the solvent. Examples of the solvent include water and an alcohol. Examples of the alcohol include methanol, ethanol, 1-propanol and 2-propanol. Two or more kinds of solvents may be mixed to be used.

The solid including the silicon oxide and the template agent can be obtained through Step A. Step A usually includes a solvent removing step. The obtained solid including the silicon oxide and the template agent can be taken out by filtering, decantation, drying, centrifugal separation, a combination thereof, and the like. The mixing in Step A is preferably carried out from 0 to 300°C over 30 minutes to 1000 hours. In one aspect, the mixing may be carried out from 20 to 100°C, the mixing may be carried out at the boiling point of the solvent, the mixing may be carried out from 20 to 60°C, and the mixing may be carried out from 20 to 40°C. In one aspect, the mixing may be carried out over 30 minutes to 24 hours and the mixing may be carried out over 2 to 24 hours. In addition, the stirring can also be carried out during mixing.

### Template agent removing step

The template agent removing step is a step of removing the template agent from the solid obtained in Step A to obtain the solid including the silicon oxide and also sometimes referred to as Step B. By carrying out Step B, the solid which does not include the template agent or does not substantially include the template agent can be obtained.

The content of the template agent in the solid obtained in Step B is preferably 10% by mass or less and more preferably 1% by mass or less

The removal of the template agent can be achieved by calcining the solid including the template agent from 300 to 800°C under air or extracting it with a solvent. The template agent is preferably removed by extracting.

Whitehurst et al. have reported a technique of extracting a template agent with a solvent (see U.S. Patent No. 5143879). Any solvent may be used as long as the solvent can dissolve the compound used as the template agent. Generally, compounds which are in a liquid state at normal temperature and have 1 to 12 carbon atoms or a mixture of two or more kinds of these compounds can be used. Examples of the suitable solvent include an alcohol, a ketone, an acyclic ether or ester and a cyclic ether or ester. Examples of the alcohol include methanol, ethanol, ethylene glycol, propylene glycol, 1-propanol, 2-propanol, 1-butanol and octanol. Examples of the ketone include acetone, diethyl ketone, methyl ethyl ketone and methyl isobutyl ketone. Examples of the ether include diisobutyl ether and tetrahydrofuran. Examples of the ester include methyl acetate, ethyl acetate, butyl acetate and butyl propionate.

From the viewpoint of dissolution ability of the template agent, for example, when the template agent is a compound including the quaternary ammonium ion, an alcohol is preferable as the solvent, and among those, methanol is more preferable. The mass ratio of the solvent to the solid including the template agent is usually from 1 to 1000 and preferably from 5 to 300. An acid or a salt thereof may be added to these solvents in order to improve the extraction effect. Examples of the acid used include an inorganic acid such as hydrochloric acid, sulfuric acid, nitric acid and hydrobromic acid, or an organic acid such as formic acid, acetic acid and propionic acid. Examples of the salt thereof include an alkali metal salt, an alkaline earth metal salt and an ammonium salt. The concentration of the acid or the salt thereof added in the solvent is preferably 30% by mass or less and even more preferably 15% by mass or less.

Examples of the template agent removing method include a method in which the solvent is sufficiently mixed with the solid including the template agent and then the liquid phase part is separated through a method such as filtering, decantation, drying, centrifugal separation, and a combination thereof. This operation may be repeated multiple times. It is also possible to extract the template agent through a method in which a container such as a column is filled with the solid including the template agent and an extraction solvent is passed therethrough. The extraction temperature is preferably from 0 to 200°C and even more preferably from 20 to 100°C. When the boiling point of the extraction solvent is low, the extraction may be carried out under pressure.

Carrying out a treatment such as an ion exchange if necessary, allows the template agent in the solution obtained by the extract treatment to be recovered to be reused as the template agent in Step A. In addition, similarly, the purification by a normal distillation operation and the like also allows the extraction solvent to be reused.

### Silylation step

The silylation step is a step of contacting the solid obtained in Step B with a silylating agent to obtain a solid including a silylated silicon oxide and also sometimes referred to as Step C. By carrying out Step C, the silicon oxide included in the solid obtained in Step B is silylated.

The silylation may be carried out through a gas phase method in which a gaseous silylating agent is contacted and reacted with the solid obtained in Step B or may be carried out through a liquid phase method in which a silylating agent is contacted and reacted with the solid in a solvent. In one aspect of the present invention, the liquid phase method is more preferable. Usually, when the silylation is carried out through the liquid phase method, a hydrocarbon is suitably used in Step C as a solvent. When the silylation is carried out through the liquid phase method, the drying may be carried out thereafter.

The silylating agent is a silicon compound having reactivity with the solid and a hydrolytic group is bonded to silicon. Examples of the hydrolytic group bonded to silicon include hydrogen, halogen, an alkoxy group, an acetoxy group and an amino group. In the silylating agent, the hydrolytic group bonded to silicon is preferably one. In addition, at least one or more groups selected from the group consisting of an alkyl group, an allyl group such as a vinyl group, an aryl group such as a phenyl group, a halogenated alkyl group, a siloxy group, and the like are bonded to silicon.

Examples of the silylating agent include an organic silane, an organic silylamine, an organic silylamide and a derivative thereof, and an organic silazane.

Examples of the organic silane include chlorotrimethylsilane, dichlorodimethylsilane, chlorobromodimethylsilane, nitrotrimethylsilane, chlorotriethylsilane, iododimethylbutylsilane, chlorodimethylphenylsilane, chlorodimethylsilane, dimethyl-n-propylchlorosilane, dimethylisopropylchlorosilane, tert-butyldimethylchlorosilane, tripropylchlorosilane, dimethyloctylchlorosilane, tributylchlorosilane, trihexylchlorosilane, dimethylethylchlorosilane, dimethyloctadecylchlorosilane, n-butyldimethylchlorosilane, bromomethyldimethylchlorosilane, chloromethyldimethylchlorosilane, 3-chloropropyldimethylchlorosilane, dimethoxymethylchlorosilane, methylphenylchlorosilane, methylphenylvinylchlorosilane, benzyldimethylchlorosilane, diphenylchlorosilane, diphenylmethylchlorosilane, diphenylvinylchlorosilane, tribenzylchlorosilane, methoxytrimethylsilane, dimethoxydimethylsilane, trimethoxymethylsilane, ethoxytrimethylsilane, diethoxydimethylsilane, triethoxymethylsilane, trimethoxyethylsilane, dimethoxydiethylsilane, methoxytriethylsilane, ethoxytriethylsilane, diethoxydiethylsilane, triethoxyethylsilane, methoxytriphenylsilane, dimethoxydiphenylsilane, trimethoxyphenylsilane, ethoxytriphenylsilane, diethoxydiphenylsilane, triethoxyphenylsilane, dichlorotetramethyldisiloxane, 3-cyanopropyldimethylchlorosilane, 1,3-dichloro-1,1,3,3-tetramethyldisiloxane and 1,3-dimethoxy-1,1,3,3-tetramethyldisiloxane.

Examples of the organic silylamine include N-(trimethylsilyl)imidazole, N-(tertbutyldimethylsilyl)imidazole, N-(dimethylethylsilyl)imidazole, N-(dimethyl-n-propylsilyl)imidazole, N-(dimethylisopropylsilyl)imidazole, N-(trimethylsilyl)-N,N-dimethylamine, N-(trimethylsilyl)-N,N-diethylamine, N-(trimethylsilyl)pyrrole, N-(trimethylsilyl)pyrrolidine, N-(trimethylsilyl)piperidine, 1-cyanoethyl(diethylamino)dimethylsilane and pentafluorophenyldimethylsilylamine.

Examples of the organic silylamide and the derivative thereof include N,O-bis(trimethylsilyl)acetamide, N,O-bis(trimethylsilyl)trifluoroacetamide, N-(trimethylsilyl)acetamide, N-methyl-N-(trimethylsilyl)acetamide, N-methyl-N-(trimethylsilyl)trifluoroacetamide, N-methyl-N-(trimethylsilyl)heptafluorobutylamide, N-(tertbutyldimethylsilyl)-N-trifluoroacetamide and N,O-bis(diethylhydrosilyl)trifluoroacetamide.

Examples of the organic silazane include 1,1,1,3,3,3-hexamethyldisilazane, heptamethyldisilazane, 1,1,3,3-tetramethyldisilazane, 1,3-bis(chloromethyl)-1,1,3,3-tetramethyldisilazane, 1,3-divinyl-1,1,3,3-tetramethyldisilazane and 1,3-diphenyl-1,1,3,3-tetramethyldisilazane.

Further examples of silylating agent include N-methoxy-N,O-bis(trimethylsilyl)trifluoroacetamide, N-methoxy-N,O-bis(trimethylsilyl)carbamate, N,O-bis(trimethylsilyl)sulfamate, trimethylsilyl trifluoromethanesulfonate and N,N'-bis(trimethylsilyl)urea.

The silylating agent is preferably an organic silazane and more preferably 1,1,1,3,3,3-hexamethyldisilazane.

The solid which is obtained in Step B and includes the silicon oxide contacts with the silylating agent, thereby silylating the silicon oxide. It is assumed that, in at least a part of the solid including the silicon oxide, a silyl group is introduced into an OH group on its surface to make it hydrophobized, however, the present invention is not limited to this theory.

### Titanium introducing step

The titanium introducing step is a step of introducing titanium into a system. "In a system" means "in a reaction system" in a method for producing a titanium-containing silicon oxide, and specifically, is included before Step A, in Step A, between Step A and Step B, in Step B, between Step B and Step C, in Step C, and after Step C.

Titanium is introduced into the system, thereby mixing the silicon oxide with the titanium source to introduce a bond represented by -Si-O-Ti into the silicon oxide.

Titanium may be introduced into the silicon oxide by contacting the silicon oxide with the titanium source in the liquid phase, and titanium may be introduced into the silicon oxide by contacting the silicon oxide with gas including the titanium source.

When the mixing is carried out in the liquid phase, examples of the solvent include water and an alcohol and, for example, the above-described solvents for Step A can be used. Examples of the mixing temperature include from 0 to 60°C. Examples of the mixing time include from 1 minute to 24 hours.

When the mixing is carried out in the gas phase, the titanium source can be gasified and mixed. Examples of the mixing temperature include from 100 to 500°C. Examples of the mixing time include from 1 minute to 24 hours. The mixing may be carried out at normal pressure and, for example, may be carried out at 10 to 1000 kPa (absolute pressure).

Titanium may be introduced in any of before Step A, in Step A, between Step A and Step B, in Step B, between Step B and Step C, in Step C and after Step C. Titanium may be introduced at two or more timings of the timings described above.

Titanium is preferably introduced before starting Step C, titanium is more preferably introduced in at least one or more selected from the group consisting of before Step A, in Step A and between Step B and Step C, and titanium is even more preferably introduced before Step A or in Step A.

When titanium is introduced before Step A, the titanium source is mixed with the silicon source, the template agent or the solvent before mixing in Step A.

When titanium is introduced in Step A, the silicon source, the titanium source and the template agent are mixed in Step A.

Titanium may be introduced by contacting the solid obtained in Step A with the titanium source after finishing Step A and before starting Step B.

Titanium may be introduced by contacting the solid obtained in Step B with the titanium source after finishing Step B and before starting Step C.

Examples of the titanium source include a titanium alkoxide, a chelate type titanium complex, a titanium halide and a sulfate including titanium. Examples of the titanium alkoxide include tetramethyl titanate, tetraethyl titanate, tetrapropyl titanate, tetraisopropyl titanate, tetrabutyl titanate, tetraisobutyl titanate, tetra(2-ethylhexyl)titanate and tetraoctadecyl titanate. Examples of the chelate type titanium complex include titanium(IV)oxyacetylacetonate and titanium(IV)diisopropoxybisacetylacetonate. Examples of the titanium halide include titanium tetrachloride, titanium tetrabromide and titanium tetraiodide. Examples of the sulfate including titanium include titanyl sulfate.

### Use of titanium-containing silicon oxide

The produced titanium-containing silicon oxide can be used as a catalyst for an oxidation reaction of an organic compound, for example, an epoxidation reaction of an olefin and is particularly preferably used for an epoxide production in which an olefin reacts with a hydroperoxide.

The olefin to be subjected to the epoxidation reaction may be an acyclic olefin, a monocyclic olefin, a bicyclic olefin or a polycyclic olefin having three or more rings, and may be a monoolefin, a diolefin or a polyolefin. When there are two or more double bonds in a molecule of the olefin, these double bonds may be a conjugated bond or a non-conjugated bond. The olefin having 2 to 60 carbon atoms is preferable. The olefin may have a substituent. Examples of such an olefin include ethylene, propylene, 1-butene, isobutylene, 1-hexene, 2-hexene, 3-hexene, 1-octene, 1-decene, styrene and cyclohexene. A substituent may be present in the olefin, the substituent containing an oxygen atom, a sulfur atom or a nitrogen atom together with a hydrogen atom or a carbon atom, or both of them. Examples of such an olefin include allyl alcohol, crotyl alcohol, and allyl chloride. Examples of the diolefin include butadiene and isoprene. Examples of the preferred olefin include an α-olefin. Examples of the particularly preferred olefin include propylene.

Examples of the hydroperoxide include an organic hydroperoxide. The organic hydroperoxide is a compound having formula III,

R-O-O-H III

wherein R is a hydrocarbon group. The organic hydroperoxide reacts with the olefin to generate an epoxide and a hydroxyl compound. In formula III, R is preferably a hydrocarbon group having 3 to 20 carbon atoms and more preferably a hydrocarbon group having 3 to 10 carbon atoms. R may be linear or branched, and may be aliphatic or aromatic. Specific examples of the organic hydroperoxide include tert-butyl hydroperoxide, 1-phenylethyl hydroperoxide, and cumene hydroperoxide. Hereinafter, cumene hydroperoxide is sometimes abbreviated as CMHP.

When CMHP is used as the organic hydroperoxide, the obtained hydroxyl compound is 2-phenyl-2-propanol. Undergoing a dehydration reaction and a hydrogenation reaction of this 2-phenyl-2-propanol allows cumene to be generated. Hereinafter, cumene is sometimes abbreviated as CUM. Further, this CUM is oxidized, thereby obtaining CMHP again. From the viewpoint of these, CMHP is preferably used as an organic hydroperoxide used in the epoxidation reaction.

The epoxidation reaction can be carried out using a solvent, a diluent or a mixture thereof in the liquid phase. The solvent and the diluent need to be liquid under the temperature and pressure upon reacting and be substantially inactive to reactants and products. When CMHP is subjected to the epoxidation reaction in the presence of CUM which is a raw material thereof, CUM can also be used as a solvent without adding a solvent, especially.

The temperature of the epoxidation reaction is generally from 0 to 200°C and is preferably a temperature from 25 to 200°C. The pressure of the epoxidation reaction can be a pressure sufficient to keep the reaction phase in a liquid state, and is generally preferably from 100 to 10000 kPa.

After finishing the epoxidation reaction, the liquid mixture containing a desired product can be separated from the catalyst composition. The liquid mixture can then be purified by a suitable method. Examples of the purification method include distillation, extraction and washing. The solvent and the unreacted olefin can be recirculated and reused.

The reaction using the titanium-containing silicon oxide produced according to one aspect of the present invention as a catalyst can be carried out in a form of a slurry or a fixed bed, and when a large-scale industrial operation is performed, the fixed bed is preferably used. When the titanium-containing silicon oxide produced according to one aspect of the present invention is used as a catalyst, the titanium-containing silicon oxide can be powder or a molded product. When being reacted in the fixed bed, the titanium-containing silicon oxide is preferably a molded product. This reaction can be carried out by a batch method, a semicontinuous method or a continuous method.

### EXAMPLES

Hereinafter, one aspect of the present invention will be described in further detail with reference to Examples.

### Example 1

### Raw material mixing step and titanium introducing step

1.0g of hexadecyldimethylamine (CDMA) was added to hexadecyltrimethylammonium hydroxide (CTAH) in which is diluted to a concentration of 16% by mass with a mixed solvent having a mixing ratio (mass ratio) of water:methanol = 72:28 (80.6 g in a solution amount having a concentration of 16% by mass), and the mixture was stirred for 5 minutes to prepare a template agent solution having a molar ratio of CDMA/CTAH of 0.09.

A mixed solution of 1.5 g of tetraisopropyl titanate and 3.5 g of 2-propanol was dropped thereto at room temperature under stirring. After the dropping was finished followed by stirring for 30 minutes, 30 g of tetramethyl orthosilicate was dropped under stirring. The stirring was then continued at 40°C for 90 minutes, and the resulting solid was filtered. The obtained solid was dried under reduced pressure at 70°C to obtain a white solid.

Hexadecyltrimethylammonium hydroxide and hexadecyldimethylamine are the template agents, and tetramethyl orthosilicate and tetraisopropyl titanate are the silicon source and the titanium source, respectively.

### Template agent removing step

5g of the white solid obtained in the raw material mixing step was put into a flask and 50 mL of methanol was added thereto. The mixture was continued to be heated to reflux for 1 hour while stirring and allowed to cool, and then the solid was filtered.

A mixed solution of 48 mL of methanol and 2.6 g of an aqueous solution of concentrated hydrochloric acid (the content of 36% by weight) was added to the obtained solid. The mixture was continued to be heated to reflux for 1 hour while stirring and allowed to cool, and then the solid was filtered.

The same operation was repeated once with respect to the obtained solid using a mixed solution of 49 mL of methanol and 1.3 g of an aqueous solution of concentrated hydrochloric acid (the content of 36% by weight).

50 ml of methanol was added to the obtained solid, the mixture was stirred at room temperature for 5 minutes, and then the solid was filtered.

50 ml of methanol was added to the obtained solid. The mixture was continued to be heated to reflux for 1 hour and allowed to cool, and then the solid was filtered. The obtained white solid was dried under reduced pressure at 120°C and 10 mmHg for 1.5 hours.

### Silylation step

2 g of the white solid obtained in the template agent removing step was mixed with 1.4 g of hexamethyldisilazane and 20 g of toluene and the mixture was heated to reflux for 1.5 hours while stirring. The mixture was allowed to cool and the solid was then filtered. The obtained solid was washed with 30 g of toluene and dried under reduced pressure at 120°C and 10 mmHg for 2 hours, thereby obtaining a titanium-containing silicon oxide catalyst.

### Synthesis of propylene oxide

A 200 mL autoclave made of SUS was charged with 0.10 g of the titanium-containing silicon oxide catalyst prepared as described above, 60 g of cumene solution containing 25% by weight of cumene hydroperoxide (CMHP) and 33 g of propylene, and then the epoxidation reaction was carried out in a batch reaction of the reaction temperature of 100°C and the reaction time of 90 minutes to obtain propylene oxide (PO). The reaction performance is shown in Table 1.

The conversion of CMHP and the selectivity of PO were determined as follows.
- Conversion of CMHP = M₂/M₀
   M₀: Molar amount of raw material CMHP
   M₁: Molar amount of CMHP remaining after reaction
   M₂: Molar amount of CMHP consumed (= Mo - M₁)
- Molar amount of PO generated: Mₚₒ (= M₂ - (Mₚₕ + M_{ac} + M_{pg} + 2 × M_{dpg} + 3 × M_{tpg}))
   Mₚₕ: Molar amount of phenol generated
   M_{ac}: Molar amount of acetophenone generated
   M_{pg}: Molar amount of propylene glycol generated
   M_{dpg}: Molar amount of dipropylene glycol generated
   M_{tpg}: Molar amount of tripropylene glycol generated
- Selectivity of PO = Mₚₒ/M₂
- Yield of PO = Conversion of CMHP × Selectivity of PO

### Example 2

A titanium-containing silicon oxide catalyst was obtained by performing the same operation as in Example 1 except that the amount of CDMA added was specified to 1.5 g and the template agent solution having a molar ratio of CDMA/CTAH of 0.13 was prepared in the raw material mixing step and the titanium introducing step.

The epoxidation reaction was carried out using the titanium-containing silicon oxide catalyst prepared as described above in the same manner as in Example 1. The reaction performance is shown in Table 1.

### Example 3

A titanium-containing silicon oxide catalyst was obtained by performing the same operation as in Example 1 except that the amount of CDMA added was specified to 0.5 g and the template agent solution having a molar ratio of CDMA/CTAH of 0.04 was prepared in the raw material mixing step and the titanium introducing step.

The epoxidation reaction was carried out using the titanium-containing silicon oxide catalyst prepared as described above in the same manner as in Example 1. The reaction performance is shown in Table 1.

### Example 4

A titanium-containing silicon oxide catalyst was obtained by performing the same operation as in Example 1 except that the amount of CDMA added was specified to 0.1 g and the template agent solution having a molar ratio of CDMA/CTAH of 0.01 was prepared in the raw material mixing step and the titanium introducing step.

The epoxidation reaction was carried out using the titanium-containing silicon oxide catalyst prepared as described above in the same manner as in Example 1. The reaction performance is shown in Table 1.

### Comparative Example 1

A titanium-containing silicon oxide catalyst was obtained by performing the same operation as in Example 1 except that the amount of CDMA added was specified to 0 g and the template agent solution having a molar ratio of CDMA/CTAH of 0 was prepared in the raw material mixing step and the titanium introducing step.

The epoxidation reaction was carried out using the titanium-containing silicon oxide catalyst prepared as described above in the same manner as in Example 1. The reaction performance is shown in Table 1.

### Comparative Example 2

A titanium-containing silicon oxide catalyst was obtained by performing the same operation as in Example 1 except that the amount of CDMA added was specified to 2.1 g and the template agent solution having a molar ratio of CDMA/CTAH of 0.18 was prepared in the raw material mixing step and the titanium introducing step.

The epoxidation reaction was carried out using the titanium-containing silicon oxide catalyst prepared as described above in the same manner as in Example 1. The reaction performance is shown in Table 1.

**[Table 1]**

| | Exam ple 1 | Exam ple 2 | Exam ple 3 | Exam ple 4 | Comparative Example 1 | Comparative Example 2 |
|---|---|---|---|---|---|---|
| Molar ratio of CDMA/CTAH in template agent (-) | 0.09 | 0.13 | 0.04 | 0.01 | 0 | 0.18 |
| Conversion of CMHP (%) | 83.5 | 76.4 | 85.8 | 70.7 | 63.9 | 65.5 |
| Yield of PO (mmol) | 81.5 | 74.8 | 83.9 | 69.0 | 62.7 | 63.7 |
| Selectivity of PO (%) | 99.0 | 99.4 | 99.2 | 99.0 | 99.5 | 98.6 |
| Percent Yield of PO (%) | 82.7 | 75.9 | 85.1 | 70.0 | 63.6 | 64.6 |

### INDUSTRIAL APPLICABILITY

The method for producing a titanium-containing silicon oxide according one aspect of the present invention can be applied to a production of a catalyst which can be used in a reaction of generating an epoxide from an olefin and a hydroperoxide, and the titanium-containing silicon oxide obtained by the method can be used, for example, in a production of a propylene oxide as a catalyst.

## Claims

1. A method for producing a titanium-containing silicon oxide, comprising the following steps:
a step of mixing a silicon source, a template agent and a solvent to obtain a solid including a silicon oxide and the template agent (raw material mixing step);
a step of removing the template agent from the solid obtained in the raw material mixing step to obtain a solid including a silicon oxide (template agent removing step);
a step of contacting the solid obtained in the template agent removing step with a silylating agent to obtain a solid including a silylated silicon oxide (silylation step); and
a step of introducing titanium into a system (titanium introducing step), wherein
the template agent is a mixture of a quaternary ammonium compound having a quaternary ammonium ion represented by the following formula I and a tertiary amine compound represented by the following formula II,
[NR¹R²R³R⁴]⁺ I
wherein
R¹ represents a hydrocarbon group having 2 to 36 carbon atoms; and
R², R³ and R⁴ each independently represent a hydrocarbon group having 1 to 6 carbon atoms, and
NR⁵R⁶R⁷ II
wherein
R⁵ represents a hydrocarbon group having 2 to 36 carbon atoms; and
R⁶ and R⁷ each independently represent a hydrogen atom or a hydrocarbon group having 1 to 6 carbon atoms; and
a molar ratio of the tertiary amine compound to the quaternary ammonium compound is from 0.005 to 0.16.

2. The method according to claim 1, wherein R¹ is a hydrocarbon group having 10 to 22 carbon atoms.

3. The method according to claim 1 or 2, wherein R⁵ is a hydrocarbon group having 10 to 22 carbon atoms.

4. The method according to any one of claims 1 to 3, wherein R¹ and R⁵ are the same as each other.

5. The method according to claim 1, wherein the quaternary ammonium ion is hexadecyltrimethylammonium ion, and
the tertiary amine compound is hexadecyldimethylamine.

6. The method according to any one of claims 1 to 5, wherein the molar ratio of the tertiary amine compound to the quaternary ammonium compound is from 0.01 to 0.13.

7. The method according to claim 6, wherein the molar ratio of the tertiary amine compound to the quaternary ammonium compound is from 0.04 to 0.13.

8. The method according to any one of claims 1 to 7, wherein the raw material mixing step is the following step and the titanium introducing step is carried out in the raw material mixing step:
the step of mixing a silicon source, a titanium source, a template agent and a solvent to obtain a solid including a silicon oxide into which titanium is introduced and a template agent.

9. A titanium-containing silicon oxide producible by the method according to any one of claims 1 to 8.

10. A method for producing an epoxide, comprising a step of reacting an olefin with a hydroperoxide in the presence of a titanium-containing silicon oxide producible by the method according to any one of claims 1 to 8 or the titanium-containing silicon oxide according to claim 9.

11. The method according to claim 10, wherein the olefin is an α-olefin.

12. The method according to claim 10, wherein the olefin is propylene.

13. The method according to any one of claims 10 to 12, wherein the hydroperoxide is cumene hydroperoxide.
